# EUROPEAN PATENT SPECIFICATION

(11) **EP 1 696 958 B1**
(45) Date of publication and mention of the grant of the patent: **28.03.2007**
(21) Application number: 04814825.8
(22) Date of filing: 17.12.2004
(51) Int. Cl.: A61K 45/00, A61P 27/02

(54) **AGENTS FOR TREATMENT OF GLAUCOMATOUS RETINOPATHY AND OPTIC NEUROPATHY**
MITTEL ZUR BEHANDLUNG VON GLAUKOMATÖSER RETINOPATHIE UND OPTISCHER NEUROPATHIE
AGENTS POUR TRAITER LA RETINOPATHIE GLAUCOMATEUSE ET LA NEUROPATHIE OPTIQUE

(30) Priority: 22.12.2003 US 531770 P
(43) Date of publication of application: 06.09.2006
(73) Proprietor: Alcon, Inc., 6331 Hünenberg (CH)
(72) Inventor: LANDERS, Robert, A., Arlington, Texas 76012 (US); PANG, Iok-Hou, Grand Prairie, Texas 75052 (US)
(74) Representative: Gates, Marie Christina Esther
(86) International application number: PCT/US2004/042687
(87) International publication number: WO 2005/063295

(56) References cited:
- EP-A- 0 742 012
- WO-A-03/028715
- WO-A-03/051313
- WO-A-03/068202
- WO-A-20/04012677
- US-A- 6 103 756
- DATABASE WPI Section Ch, Week 199943, 1999 Derwent Publications Ltd., London, GB; Class B02, AN 1999-518727 XP002318667 YOSHOYUKI: "Therapeutic agent for complication of diabetes" & WO 99 43315 A (SHIONOGI), 2 September 1999 (1999-09-02)
- DATABASE WPI Section Ch, Week 199750, 1997 Derwent Publications Ltd., London, GB; Class B02, AN 1997-539728 XP002318668 GODO: "Suppressant for maillard reaction" & JP 09 241165 A (SNOW BRAND MILK PROD), 16 September 1997 (1997-09-16)

## Description

### FIELD OF THE INVENTION

The present invention relates to the field of prophylactic agents and therapeutics for retinopathy and optic neuropathy related to glaucoma.

### BACKGROUND OF THE INVENTION

Glaucoma is a heterogeneous group of diseases that have a similar set of clinical features including optic nerve damage and selective apoptotic death of retinal ganglion cells (RGC), which leads to a progressive loss of visual field and blindness. An abnormal increase in intraocular pressure (IOP) is associated with most forms of glaucoma. The only available treatment is to lower IOP either by medication or surgery. Lowering IOP is effective in slowing the development of certain types of glaucoma and delaying its damaging effects. Nonetheless, the loss of visual field in glaucoma patients does not always correlate with IOP, and lowering IOP alone does not completely stop the disease process. This implicates that pressure may not be the only cause of glaucomatous retinopathy and optic neuropathy. Additional mechanisms likely contribute to the disease processes. Glaucomatous retinopathy is generally understood as functional disturbances or pathological changes in the retina, especially the death of RGC, that are found in patients or animals with glaucoma. Glaucomatous optic neuropathy refers to functional disturbances or pathological changes in the optic nerve, through which axons of RGC pass.

A cross-section through the adult human retina shows the following layers of cells listed in the direction from the proximal or innermost region (vitreous side) to the distal or outermost region (choroidal side):
inner limiting membrane,
optic fiber layer,
ganglion cell layer,
inner plexiform layer,
inner nuclear layer,
outer plexifonn layer,
outer nuclear layer,
external limiting membrane,
inner segments of rods and cones,
outer segments of rods and cones,
retinal pigment epithelium, and
choriocapillaris.

The retinal pigment epithelium and choriocapillaries are found at the back of the retina closest to the choroid membrane, while the inner limiting membrane is closest to the vitreal chamber. The ganglion cell layer collects photoreceptive input and sends the input via myelinated axons through the optic nerve to the brain. The ganglion cells are the cells at risk in glaucoma.

Molecular mechanisms proposed for contributing to the death of RGC include glutamate toxicity, withdrawal of neurotrophic factors, vascular abnormality (ischemia), reactive gliosis, and nitric oxide-induced toxicity. However, none of these proposed mechanisms is universally accepted by researchers in the field.

PCT patent application no. PCT/US02/40457 to Gao, X., *et al.,* published as WO 03/051313, reportedly provides an induction of a phase II detoxification enzyme by sulforaphane in human retinal pigment epithelial cells. U.S. Patent Publication No. 2002/0091087, to Zhang, Y., *et al.*, reportedly provides treatment of a neurodegenerative disease via a compound, sulforaphane, that elevates glutathione or a Phase II detoxification enzyme in spinal cord tissue, Alzheimer's disease, and in amyotrophic lateral sclerosis. Retinal pigment epithelial cells differ from retinal ganglion cells in that the ganglion cells are neurons and the retinal pigment epithelial cells are not neurons. Further, biological responses of ocular tissues such as the retina to particular therapeutic agents cannot be predicted from the biological responses of spinal cord tissues and brain tissues. The cited applications do not address protection or treatment for loss of retinal ganglion cells (RGC) and optic neuropathy in glaucoma.

There is no generally accepted anti-glaucoma therapeutic method to manage glaucomatous retinopathy and optic neuropathy. In view of the impact of glaucoma on health, and the inadequacies of prior methods of treatment, it would be desirable to have an improved method of treatment that addresses glaucomatous retinopathy and optic neuropathy.

### SUMMARY OF THE INVENTION

According to the present invention, an agent having stimulatory activity for Nrf2 protein nuclear translocation and the subsequent increases in gene products that detoxify and eliminate cytotoxic metabolites provides a protective or therapeutic effect in delaying or preventing loss of retinal ganglion cells and glaucomatous damage to the optic nerve. As used herein "stimulatory activity for Nrf2 protein nuclear translocation" means an agent that enhances the availability or the transport of Nrf2 to the nucleus. Translocation of Nrf2 protein to the nucleus allows a subsequent increase in expression of gene products that detoxify and eliminate cytotoxic metabolites. The methods of the present invention provide a method of treatment for glaucomatous retinopathy and optic neuropathy in a subject comprising administering to the subject an effective amount of a composition comprising an agent having stimulatory activity for Nrf2 protein nuclear translocation, and an acceptable carrier. The subject may be at risk for developing glaucomatous retinopathy or optic neuropathy or may have symptoms of glaucomatous retinopathy or optic neuropathy.

The agent that stimulates nuclear translocation of Nrf2 protein and the subsequent increases in gene products that detoxify and eliminate cytotoxic metabolites of the present invention may comprise a Michael Addition acceptor, diphenol, thiocarbamate, quinone, 1,2-dithiole-3-thione, butylated hydroxyanisole, flavonoid, an isothiocyanate, 3,5-di-tert-butyl-4-hydroxytoluene, ethoxyquin, 3-hydroxycoumarin, combinations thereof, or a pharmacologically active derivative or analog thereof. In one embodiment, the agent comprises an isothiocyanate such as sulforaphane, or a pharmacologically active derivative thereof. In another embodiment, the agent comprises a 1,2-dithiole-3-thione such as oltipraz, or a pharmacologically active derivative thereof.

Administration of the agent that stimulates nuclear translocation of Nrf2 protein and the subsequent increases in gene products that detoxify and eliminate cytotoxic metabolites may be by intraocular injection, implantation of a slow release delivery device, or topical, oral, intranasal administration, systemic injection, or other systemic administrations.

In a further embodiment of the present inventive method, the subject is diagnosed with glaucomatous retinopathy or optic neuropathy and, in another embodiment of the invention, the subject has symptoms of glaucomatous retinopathy or optic neuropathy.

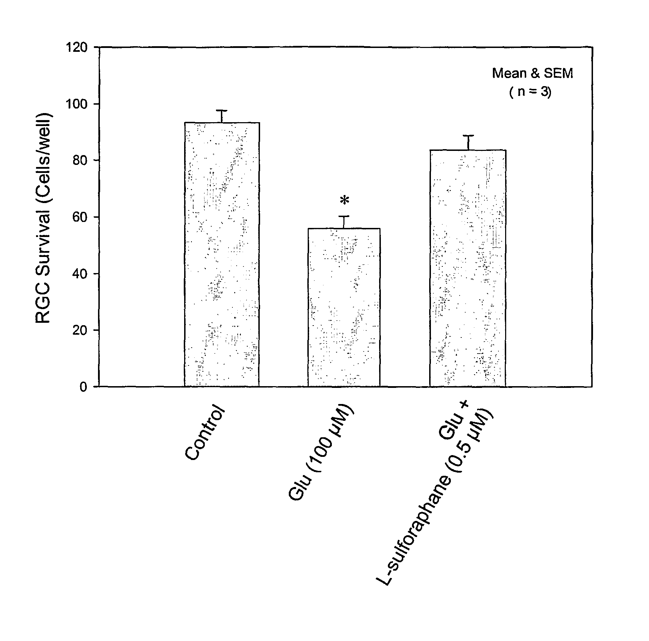

### BRIEF DESCRIPTION OF THE DRAWING

The drawing demonstrates the effect of sulforaphane on glutamate-induced toxicity in cultured adult rat retinal ganglion cells. Cells were treated with the indicated compounds for 3 days. Survival was assayed by counting Thy-1-positive healthy cells. The asterisk * represents a significant difference from the control values by one-way ANOVA analysis of variance between groups, then Dunnett's test.

### DETAILED DESCRIPTION OF THE INVENTION

The present invention relates to use of agents that stimulates nuclear translocation of Nrf2 protein and the subsequent increases in gene products that detoxify and eliminate cytotoxic metabolites in a composition for treating glaucomatous retinopathy and optic neuropathy.

The term "treating glaucomatous retinopathy and optic neuropathy," as used herein, means delaying or preventing the development of, inhibiting the progression of, or alleviating glaucomatous retinopathy or optic neuropathy, or symptoms thereof. Stimulating nuclear translocation of Nrf2 protein and the subsequent increases in gene products that detoxify and eliminate cytotoxic metabolites is provided for protection of retinal ganglion cells and for protection of the optic nerve.

The nuclear translocation of Nrf2 is induced in cells exposed to certain electrophiles and oxidants. Genes induced due to nuclear translocation of Nrf2 yield detoxification enzymes that enhance protection against electrophiles and promote the repair or degradation of damaged proteins. Induction of these enzymes is regulated at the transcriptional level and is mediated by a specific enhancer, the antioxidant response element or ARE, found in the promoter of the gene encoding the enzyme. The sequence context of the ARE, the nature of the chemical inducers, and the cell type affect the activity of the enhancer in a particular gene.

The transcription factor Nrf2 is a member of the NF-E2 transcription factor family and is responsible for upregulating the antioxidant response element (ARE)-mediated gene expression. Nrf2 induces gene expression by binding to the ARE (antioxidant response element) region of the promoter to activate gene transcription constitutively or in response to an oxidative stress signal. Under normal conditions, Nrf2 is thought to be present in the cytoplasm bound by a repressor protein Keap1, a cytoplasmic protein anchored to the actin cytoskeleton. Not wanting to be bound by theory, the inventors believe that agents having stimulatory activity for Nrf2 protein nuclear translocation may compete with the cysteine-rich intervening region of a cytosolic factor Keap1 for interaction with Nrf2 (Dinlcova-Kostova, A.T., *et al., Proc Natl Acad Sci,* USA, 99:11908-11913 (2002)). Disruption of the Nrf2-Keap1 complex by certain compounds such as sulforaphane may free Nrf2 to translocate into the nucleus where it can heterodimerize with other transcription factors (*i.e.*, Maf, c-Jun, etc.) on ARE regions of genes leading to induction of ARE-regulated gene expression.

Enzymes and proteins expressed by this Nrf2/ARE pathway possess chemically versatile cytoprotective properties and are a defense against toxic metabolites and xenobiotics. Enzymes and proteins known to be expressed through the Nrf2/ARE pathway include glutathione-*S*-transferases, UDP-glucuronosyltransferases, NADP(H) quinone oxidoreductase, γ-glutamylcysteine synthetase, chaperone/stress response proteins, and ubiquitin/proteasome proteins.

Agents having stimulatory activity for Nrf2 protein nuclear translocation include, for example:
Michael addition acceptors (e.g., α,β-unsaturated carbonyl compounds), such as diethyl maleate or dimethylfumarate;
diphenols such as resveratrol,
butylated hydroxyanisoles such as 2(3)-tert-butyl-4-hydroxyanisole,
thiocarbamates such as pyrrolidinedithiocarbamate,
quinones such as tert-butyl-hydroquinone,
isothiocyanates such as sulforaphane, its precursor glucosinolate, glucoraphanin, or phenethyl isothiocyanate (PEITC),
1,2-dithiole-3-thiones such as oltipraz,
3,5-di-tert-butyl-4-hydroxytoluene,
ethoxyquin,
coumarins such as 3-hydroxycoumarin,
flavonoids such as quercetin or curcumin,
diallyl sulfide,
indole-3-carbinol,
epigallo-3-catechin gallate,
ellagic acid,
combinations thereof, or a pharmacologically active derivative or analog thereof.

A Michael acceptor is a molecule that has an alkene adjacent to an electron withdrawing group. The electron withdrawing group is usually a carbonyl, but can also be a nitrile or nitro group. Though chemically diverse, these compounds are electrophiles and have the ability to react with nucleophilic sulfhydryl groups. A "pharmacologically active derivative thereof," is an agent structurally related to any of the above compounds having stimulatory activity for Nrf2 protein nuclear translocation and derivable from it and may be an ester, an amide, or a salt thereof, for example. A "pharmacologically active analog thereof," is an agent that is structurally similar to any of the above compounds having stimulatory activity for Nrf2 protein nuclear translocation but differs slightly in composition such as in the replacement of one atom by an atom of a different element or in the presence of a particular functional group, for example. In one embodiment, the present invention provides sulforaphane, oltipraz, a pharmacologically active analog thereof, or a pharmaceutically acceptable salt thereof in a method of treatment for glaucomatous optic neuropathy or glaucomatous retinopathy.

Sulforaphane (Product no. S6317, Sigma-Aldrich) is known to induce quinone reductase, glutathione-*S*-transferase, and glutathione reductase, for example. Enzyme induction has been observed in various cell lines including human adult retinal pigment epithelial cells (Zhang, Y. *et al., Proc Natl Acad Sci,* USA, 89:2399-2403 (1992)). Sulforaphane analogs include, for example, 6- (isothiocyanato-2-hexanone), exo-2-acetyl-6-isothiocyanatonorbomane, exo-2-(isothiocyanato-6-methylsulfonylnorbomane), 6-isothiocyanato-2-hexanol, 1- (isothiocyanato-4-dimethylphosphonylbutane, exo-2-(1-hydroxyethyl)-5-) isothiocyanatonorbornane, exo-2-acetyl-5-isothiocyanatonorbomane, 1-(isothiocyanato-5-methylsulfonylpentane), cis-3- (methylsulfonyl)(cyclohexylmethylisothiocyanate) and trans-3- (methylsulfonyl)(cyclohexylmethylisothiocyanate).

*Mode of administration:* The agents of the present invention may be delivered directly to the eye (for example: topical ocular drops or ointments; slow release devices in the cul-de-sac or implanted adjacent to the sclera or within the eye; periocular, conjunctival, sub-tenons, intracameral, intravitreal, or intracanalicular injections) or systemically (for example: orally, intravenous, subcutaneous or intramuscular injections; parenterally, dermal or nasal delivery) using techniques well known by those skilled in the art. It is further contemplated that the agents of the invention may be formulated in intraocular insert or implant devices.

*Subject:* A subject treated for glaucomatous retinopathy or optic neuropathy as described herein may be a human or another animal at risk of developing glaucomatous retinopathy or optic neuropathy or having symptoms of glaucomatous retinopathy or optic neuropathy.

*Formulations and Dosage:* The agents of the present invention can be administered as solutions, suspensions, or emulsions (dispersions) in a suitable ophthalmic carrier. The following are examples of possible formulations embodied by this invention.

| | Amount in weight % |
|---|---|
| Agent stimulating Nrf2 protein | 0.01-5; 0.01- 2.0; 0.5 - 2.0 |
| nuclear translocation | |
| Hydroxypropylmethylcellulose | 0.5 |
| Sodium chloride | .8 |
| Benzalkonium Chloride | 0.01 |
| EDTA | 0.01 |
| NaOH/HCl | qs pH 7.4 |
| Purified water | qs 100% |

| | Amount in weight % |
|---|---|
| Agent stimulating Nrf2 protein | 0.00005 - 0.5; 0.0003 - 0.3; 0.0005 - 0.03; 0.001 |
| nuclear translocation | |
| Phosphate Buffered Saline | 1.0 |
| Benzalkonium Chloride | 0.01 |
| Polysorbate 80 | 0.5 |
| Purified water | q.s. to 100% |

| | Amount in weight % |
|---|---|
| Agent stimulating Nrf2 protein | 0.001 |
| nuclear translocation | |
| Monobasic sodium phosphate | 0.05 |
| Dibasic sodium phosphate | 0.15 |
| (anhydrous) | |
| Sodium chloride | 0.75 |
| Disodium EDTA | 0.05 |
| Cremophor EL | 0.1 |
| Benzalkonium chloride | 0.01 |
| HCl and/or NaOH | pH 7.3-7.4 |
| Purified water | q.s. to 100% |

| | Amount in weight % |
|---|---|
| Agent stimulating Nrf2 protein | 0.0005 |
| nuclear translocation | |
| Phosphate Buffered Saline | 1.0 |
| Hydroxypropyl-β-cyclodextrin | 4.0 |
| Purified water | q.s. to 100% |

In a further embodiment, the ophthalmic compositions are formulated to provide for an intraocular concentration of about 0.1-100 nanomolar (nM) or, in a further embodiment, 1-10 nM. Peak plasma concentrations of up to 20 micromolar may be achieved for systemic administration. Topical compositions are delivered to the surface of the eye one to four times per day according to the routine discretion of a skilled clinician. The pH of the formulation should be 4-9, or 4.5 to 7.4. Systemic formulations may contain about 10 mg to 1000 mg, about 10 mg to 500 mg, about 10 mg to 100 mg or to 125 mg, for example, of the agent that stimulates nuclear translocation of Nrf2 protein and the subsequent increases in gene products that detoxify and eliminate cytotoxic metabolites.

An "effective amount" refers to that amount of agent that is able to stimulate nuclear translocation of Nrf2 protein and the subsequent increases in gene products that detoxify and eliminate cytotoxic metabolites. Such induction of gene expression provides a defense against the toxicity of reactive electrophiles as well as other toxic metabolites. Therefore, an agent that stimulates nuclear translocation of Nrf2 protein and the subsequent increases in gene products that detoxify and eliminate cytotoxic metabolites is provided for protection against cytotoxicity. Such protection delays or prevents onset of symptoms in a subject at risk for developing glaucomatous optic neuropathy or glaucomatous retinopathy. The effective amount of a formulation may depend on factors such as the age, race, and sex of the subject, or the severity of the optic neuropathy, for example. In one embodiment, the agent is delivered topically to the eye and reaches the retinal ganglion cells at a therapeutic dose thereby ameliorating the retinopathy or optic neuropathy disease process.

While the precise regimen is left to the discretion of the clinician, the resulting solution or solutions are preferably administered by placing one drop of each solution(s) in each eye one to four times a day, or as directed by the clinician.

*Acceptable carriers:* An ophthalmically acceptable carrier refers to those carriers that cause at most, little to no ocular irritation, provide suitable preservation if needed, and deliver one or more agents that stimulate nuclear translocation of Nrf2 protein and the subsequent increases in gene products that detoxify and eliminate cytotoxic metabolites of the present invention in a homogenous dosage. For ophthalmic delivery, an agent that stimulates nuclear translocation of Nrf2 protein and the subsequent increases in gene products that detoxify and eliminate cytotoxic metabolites may be combined with ophthalmologically acceptable preservatives, co-solvents, surfactants, viscosity enhancers, penetration enhancers, buffers, sodium chloride, or water to form an aqueous, sterile ophthalmic suspension, solution, or viscous or semi-viscous gels or other types of solid or semisolid composition such as an ointment. Ophthalmic solution formulations may be prepared by dissolving the agent in a physiologically acceptable isotonic aqueous buffer. Further, the ophthalmic solution may include an ophthalmologically acceptable surfactant to assist in dissolving the agent. Viscosity building compounds, such as hydroxymethyl cellulose, hydroxyethyl cellulose, methylcellulose, polyvinylpyrrolidone, or the like, may be added to the compositions of the present invention to improve the retention of the compound.

In order to prepare a sterile ophthalmic ointment formulation, the agent that stimulates nuclear translocation of Nrf2 protein and the subsequent increases in gene products that detoxify and eliminate cytotoxic metabolites is combined with a preservative in an appropriate vehicle, such as mineral oil, liquid lanolin, or white petrolatum. Sterile ophthalmic gel formulations may be prepared by suspending the agent in a hydrophilic base prepared from the combination of, for example, CARBOPOL^{®}-940 (BF Goodrich, Charlotte, NC), or the like, according to methods known in the art for other ophthalmic formulations. VISCOAT^{®} (Alcon Laboratories, Inc., Fort Worth, TX) may be used for intraocular injection, for example. Other compositions of the present invention may contain penetration enhancing materials such as CREMOPHOR^{®} (Sigma Aldrich, St. Louis, MO) and TWEEN^{®} 80 (polyoxyethylene sorbitan monolaureate, Sigma Aldrich), in the event the agents of the present invention are less penetrating in the eye.

### Example 1

### Agents having Stimulatory Activity for Nrf2 Protein Nuclear Translocation

Vascular endothelial cells, such as bovine aortic endothelial cells (BAEC, VEC Technologies, Rensselaer, NY), are used to determine those agents having stimulatory activity for Nrf2 protein nuclear translocation. For example, confluent monolayers of bovine aortic endothelial cells are exposed to candidate agents in Dulbecco's modified Eagle's medium with 1% fetal bovine serum for up to 24 hours. Cell lysates, cytosolic extracts, and nuclear extracts are prepared, and immunoblotting performed and quantified as described in Buckley, B.J., *et al.* (*Biochem Biophys Res Commun,* 307:973-979 (2003)). Agents that increase the amount of Nrf2 detected in the nuclear fraction as compared to control cells without agent are then tested for activity in a retinal ganglion cell toxicity assay as set forth in Example 2.

### Example 2

### Protection of Rat Retinal Ganglion Cells by an Agent Having Stimulatory Activity for Nrf2 Protein Nuclear Translocation

Cultured rat neural retinal cells are combined with an agent that stimulates nuclear translocation of Nrf2 protein for 1 to 24 hours, then the combination is exposed to peroxide. Survival of the neural retinal cells as compared to a control culture without peroxide indicates that the agent provides protection from the oxidant.

Neonatal rat neural retinal cells are isolated and cultured as reported in Pang, I-H., *et al,* (*Invest Ophthalmol Vis Sci* 40:1170-1176 (1999)). "Neural retinal" refers to the retina without the retinal pigment epithelium. Thus, the culture contains a mixed population of retinal cell types. Briefly, neonatal Sprague-Dawley rats 2-5 days old are anesthetized and a 2 mm midline opening is made in the scalp just caudal to the transverse sinus. Retinal ganglion cells are selectively retrograde labeled with a fluorescent dye, Di-I, (1,1'-dioctadecyl-3,3,3',3'-tetramethylindocarbocyanine perchlorate), a lipophilic tracer that uniformly labels neurons (Molecular Probes, Eugene, OR). The tip of the injection needle (30 ga) is inserted 6 mm below the top of the skull, and a 5 µl Di-I solution injected into the superior colliculi. The Di-I solution contains 3 mg/mL Di-I in 90% ethanol and 10% dimethylsulfoxide. The wound is covered with a drop of flexible collodion.

Two to 4 days after Di-I injection, rats are anesthetized and sacrificed by decapitation. Their eyes are enucleated and placed in Dulbecco's modified Eagle's medium:Nutrient mixture F12 (1:1; DMEM/F12, Gibco, Gaithersburg, MD). The retina from each eye is detached and isolated. Retinal cells are dissociated by a solution containing 10 mg papain (34 units/mL), 2 mg DL-cysteine (3.3 mM) and 2 mg bovine serum albumin (0.4 mg/mL) in 5 ml of DMEM/F12, for 25 min at 37°C, and then washed 3 times with 5 mL RGC medium (DMEM, supplemented with 10% fetal bovine serum, 4 mM glutamine, 100 units/mL penicillin and 100 µg/ml streptomycin). Retinal pieces are triturated by passing through a disposable pipet several times until cells are dispersed. The cell suspensions (approximately 3 x 10⁶ cells/mL) are placed into poly-D-lysine coated glass bottom culture dishes. The cells are cultured with 95% air/5% CO₂ at 37°C.

Protective effects of an agent having stimulatory activity for Nrf2 protein nuclear translocation are determined by treating the cultures with the candidate agent for 1 to 24 hours, then 300 µM of H₂O₂ are added. Cultured RGCs are identified by Di-I fluorescence. Survival of RGC is evaluated by counting the number of remaining cells with Di-I fluorescence. Agents that improve the survival of retinal ganglion cells as compared to a control are provided for protecting the RGC's from cytotoxic insult and are useful for the treatment of glaucomatous optic neuropathy.

### Example 3

### Protection of Rat Retinal Ganglion Cells from Glutamate-Induced Toxicity by Sulforaphane

Adult Sprague-Dawley rats were euthanized by CO₂ asphyxiation. Their eyes were enucleated and placed in NEUROBASAL^{™} medium (Gibco, Gaithersburg, MD). The retina from each eye was detached and isolated. Retinal cells were dissociated by combining up to 20 retinae with 5 mL of papain solution containing 10 mg papain, 2 mg DL-cysteine, and 2 mg bovine serum albumin in 5 ml of NEUROBASAL^{™} medium, for 25 min at 37°C, then washed 3 times with 5 mL RGC medium (NEUROBASAL^{™}) medium supplemented as cited in Example 2 and with 1% fetal calf serum. Retinal pieces were triturated by passing through a fire-polished disposable pipet several times until cells were dispersed. The cell suspension was placed onto a poly-D-lysine- and laminin-coated 8-well chambered culture slide. Glutamate and glutamate with sulforaphane were added to assigned wells. The cells were then cultured at 95% air/5% CO₂ at 37°C for three days.

At the end of the incubation period, the cells were fixed and labeled for Thy-1, a RGC marker, by immunocytochemistry. Cell survival was quantified by manually counting Thy-1-positive healthy cells in each well. The resulting data are shown in the drawing and demonstrate that treatment of the RGC with glutamate (100 µM) for 3 days caused a 40-60% reduction of the surviving cells. Treating the cells with sulforaphane (0.5 µM) prevented such toxicity. These results demonstrate that sulforaphane is protective against insults to the retinal ganglion cells.

The references cited herein, to the extent that they provide exemplary procedural or other details supplementary to those set forth herein, are specifically incorporated by reference.

As used herein and unless otherwise indicated, the terms "a" and "an" are taken to mean "one", "at least one" or "one or more".

## Claims

1. The use, in the preparation of a medicament for the treatment for glaucomatous retinopathy or optic neuropathy in a subject, of an effective amount of a composition comprising an agent having stimulatory activity for nuclear translocation of Nrf2 protein, and an acceptable carrier.

2. The use of claim 1 wherein the subject is at risk for developing glaucomatous retinopathy or optic neuropathy.

3. The use of claim 1 wherein the subject has symptoms of glaucomatous retinopathy or optic neuropathy.

4. The use of claims 1 to 3 wherein the agent comprises a Michael Addition acceptor, diphenol, thiocarbamate, quinone, 1,2-dithiole-3-thione, butylated hydroxyanisole, flavonoid, an isothiocyanate, 3,5-di-tert-butyl-4-hydroxytoluene, ethoxyquin, a coumarin, combinations thereof, or a pharmacologically active derivative or analog thereof.

5. The use of claim 4 wherein the agent comprises an isothiocyanate, or a pharmacologically active derivative thereof.

6. The use of claim 5 wherein the isothiocyanate comprises sulforaphane, or a pharmacologically active derivative thereof.

7. The use of claim 4 wherein the agent comprises a 1,2-dithiole-3-thione, or a pharmacologically active derivative thereof.

8. The use of claim 7 wherein the 1,2-dithiole-3-thione comprises oltipraz, or a pharmacologically active derivative thereof.

9. The use of claim 4 wherein the agent comprises a flavonoid, or a pharmacologically active derivative thereof.

10. The use of claim 9 wherein the flavonoid comprises quercetin, or a pharmacologically active derivative thereof.

11. The use of any preceding claim wherein the medicament is prepared for intraocular injection, for implantation of a slow release delivery device, or for topical, oral, or intranasal administration.

12. The use of claim 11 wherein the medicament is prepared for intraocular administration.

## Patentansprüche

1. Verwendung einer wirksamen Menge einer Zusammensetzung, umfassend ein Mittel mit stimulatorischer Aktivität hinsichtlich der nukleären Translokation von Nrf2-Protein und einen annehmbaren Träger, zur Herstellung eines Medikaments für die Behandlung von Glaukom-Retinopathie oder Optikus-Neuropathie bei einem Individuum.

2. Verwendung nach Anspruch 1, wobei bei dem Individuum die Gefahr der Entwicklung von Glaukom-Retinopathie oder Optikus-Neuropathie besteht.

3. Verwendung nach Anspruch 1, wobei das Individuum Symptome von Glaukom-Retinopathie oder Optikus-Neuropathie aufweist.

4. Verwendung nach den Ansprüchen 1 bis 3, wobei das Mittel einen Michael-Additions-Akzeptor, Diphenol, Thiocarbamat, Chinon, 1,2-Dithiol-3-thion, butyliertes Hydroxyanisol, Flavonoid, ein Isothiocyanat, 3,5-Di-tert.-butyl-4-hydroxytoluol, Ethoxychin, ein Cumarin, Kombinationen davon oder ein pharmakologisch aktives Derivat oder Analogon davon umfasst.

5. Verwendung nach Anspruch 4, wobei das Mittel ein Isothiocyanat oder ein pharmakologisch aktives Derivat davon umfasst.

6. Verwendung nach Anspruch 5, wobei das Isothiocyanat Sulforaphan oder ein pharmakologisch aktives Derivat davon umfasst.

7. Verwendung nach Anspruch 4, wobei das Mittel ein 1,2-Dithiol-3-thion oder ein pharmakologisch aktives Derivat davon umfasst.

8. Verwendung nach Anspruch 7, wobei das 1,2-Dithiol-3-thion Oltipraz oder ein pharmakologisch aktives Derivat davon umfasst.

9. Verwendung nach Anspruch 4, wobei das Mittel ein Flavonoid oder ein pharmakologisch aktives Derivat davon umfasst.

10. Verwendung nach Anspruch 9, wobei das Flavonoid Quercetin oder ein pharmakologisch aktives Derivat davon umfasst.

11. Verwendung nach irgendeinem vorhergehenden Anspruch, wobei das Medikament für die intraokulare Injektion, für die Implantation einer Abgabevorrichtung zur langsamen Freisetzung oder für die topische, orale oder intranasale Verabreichung bereitet wird.

12. Verwendung nach Anspruch 11, wobei das Medikament für die intraokulare Verabreichung bereitet wird.

## Revendications

1. Utilisation, dans la préparation d'un médicament destiné au traitement de la rétinopathie ou de la neuropathie optique glaucomateuses chez un sujet, d'une quantité efficace d'une composition comprenant un agent présentant une activité stimulatrice pour la translocation nucléaire de la protéine Nrf2 et d'un excipient acceptable.

2. Utilisation selon la revendication 1, dans laquelle le sujet présente un risque de développer une rétinopathie ou une neuropathie optique glaucomateuses.

3. Utilisation selon la revendication 1, dans laquelle le sujet présente des symptômes de rétinopathie ou de neuropathie optique glaucomateuses.

4. Utilisation selon les revendications 1 à 3, dans laquelle l'agent comprend un accepteur de Michael (addition de type Michael), un diphénol, un thiocarbamate, une quinone, une 1,2-dithiole-3-thione, un hydroxyanisole butylé, un flavonoïde, un isothiocyanate, un 3,5-di-tert-butyl-4-hydroxytoluène, une éthoxyquine, une coumarine, des combinaisons de ceux-ci ou un dérivé pharmacologiquement actif ou analogue de ceux-ci.

5. Utilisation selon la revendication 4, dans laquelle l'agent comprend un isothiocyanate ou un dérivé pharmacologiquement actif de celui-ci.

6. Utilisation selon la revendication 5, dans laquelle l'isothiocyanate comprend un sulforaphane ou un dérivé pharmacologiquement actif de celui-ci.

7. Utilisation selon la revendication 4, dans laquelle l'agent comprend une 1,2-dithiole-3-thione ou un dérivé pharmacologiquement actif de celle-ci.

8. Utilisation selon la revendication 7, dans laquelle une 1,2-dithiole-3-thione comprend de l'oltipraz ou un dérivé pharmacologiquement actif de celui-ci.

9. Utilisation selon la revendication 4, dans laquelle l'agent comprend un flavonoïde ou un dérivé pharmacologiquement actif de celui-ci.

10. Utilisation selon la revendication 9, dans laquelle le flavonoïde comprend une quercétine ou un dérivé pharmacologiquement actif de celle-ci.

11. Utilisation selon l'une quelconque des revendications précédentes, dans laquelle le médicament est préparé en vue d'une injection intraoculaire, d'une implantation d'un dispositif à libération lente ou d'une administration intranasale, orale ou topique.

12. Utilisation selon la revendication 11, dans laquelle le médicament est préparé en vue d'une administration intraoculaire.
